# EUROPEAN PATENT SPECIFICATION

(11) **EP 1 795 612 B1**
(45) Date of publication and mention of the grant of the patent: **26.12.2012**
(21) Application number: 05774925.1
(22) Date of filing: 19.08.2005
(51) Int. Cl.: C12Q 1/68, C12N 15/00, C12M 1/00, G01N 33/53, G01N 33/543, G01N 33/566

(54) **NUCLEIC ACID ANALYSIS METHOD**
NUKLEINSÄUREANALYSEVERFAHREN
PROCEDE D'ANALYSE D'ACIDE NUCLEIQUE

(30) Priority: 31.08.2004 JP 2004252767
(43) Date of publication of application: 13.06.2007
(73) Proprietor: EIKEN KAGAKU KABUSHIKI KAISHA, Tokyo 110-8408 (JP)
(72) Inventor: Mori, Yasuyoshi, c/o Nasu Kojo, Eiken Kagaku Kabushiki Kaisha, Otawara-shi, Tochigi 324-0036 (JP); Hirano, Takashi, c/o Nasu Kojo, Eiken Kagaku Kabushiki Kaisha, Otawara-shi, Tochigi 324-0036 (JP)
(74) Representative: Schrell, Andreas
(86) International application number: PCT/JP2005/015493
(87) International publication number: WO 2006/025264

(56) References cited:
- EP-A- 1 416 047
- WO-A2-03/078659
- JP-A- 2004 141 159
- JP-A- 2004 154 143
- JP-A- 2004 201 607
- JP-A- 2004 201 607
- JP-A- 2004 290 149
- MITRA R D ET AL: "In situ localized amplification and contact replication of many individual DNA molecules.", NUCLEIC ACIDS RESEARCH 15 DEC 1999 LNKD- PUBMED:10572186, vol. 27, no. 24, 15 December 1999 (1999-12-15), page e34, ISSN: 1362-4962

### SEQUENCE LISTING

<110> EIKEN KAGAKU KABUSHIKI KAISHA
<120> Method for nucleic acid analysis
<130> PH-2548-PCT
<150> JP 2004-252767
   <151> 2004-08-31
<160> 21
<170> PatentIn version 3.1
<210> 1
   <211> 44
   <212> DNA
   <213> Artificial
<220>
   <223> FIP primer for PSA
<400> 1
   tgttcctgat gcagtgggca gctttagtct gcggcggtgt tctg 44
<210> 2
   <211> 45
   <212> DNA
   <213> Artificial
<220>
   <223> RIP primer for PSA
<400> 2
   tgctgggtcg gcacagcctg aagctgacct gaaatacctg gcctg 45
<210> 3
   <211> 18
   <212> DNA
   <213> Artificial
<220>
   <223> F3 primer for PSA
<400> 3
   tgcttgtggc ctctcgtg 18
<210> 4
   <211> 17
   <212> DNA
   <213> Artificial
<220>
   <223> R3 primer for PSA
<400> 4
   gggtgtgtga agctgtg 17
<210> 5
   <211> 40
   <212> DNA
   <213> Artificial
<220>
   <223> FIP primer for CK20
<400> 5
   caatttgcag gacacaccga gattgaagag ctgcgaagtc 40
<210> 6
   <211> 40
   <212> DNA
   <213> Artificial
<220>
   <223> RIP primer for CK20
<400> 6
   ctgctgagga cttcagactg acttggagat cagcttccac 40
<210> 7
   <211> 23
   <212> DNA
   <213> Artificial
<220>
   <223> F3 primer for CK20
<400> 7
   cgactacagt gcatattaca gac 23
<210> 8
   <211> 22
   <212> DNA
   <213> Artificial
<220>
   <223> R3 primer for CK20
<400> 8
   gtagggttag gtcatcaaag ac 22
<210> 9
   <211> 21
   <212> DNA
   <213> Artificial
<220>
   <223> LpF primer for CK20
<400> 9
   gcagttgagc atccttaatc t 21
<210> 10
   <211> 20
   <212> DNA
   <213> Artificial
<220>
   <223> LpR primer for CK20
<400> 10 20
   gactgagaga ggaatacgtc 20
<210> 11
   <211> 43
   <212> DNA
   <213> Artificial
<220>
   <223> FIP primer for HCV
<400> 11 43
   ggttkatcca agaaaggacc cagtcgccat agtggtctgc gga 43
<210> 12
   <211> 38
   <212> DNA
   <213> Artificial
<220>
   <223> RIP primer for HCV
<400> 12 38
   ccgcaagact gctagccgag gcaagcaccc tatcaggc 38
<210> 13
   <211> 22
   <212> DNA
   <213> Artificial
<220>
   <223> F3 primer for HCV
<400> 13 22
   ggcgttagta tgagtgtcgt ac 22
<210> 14
   <211> 18
   <212> DNA
   <213> Artificial
<220>
   <223> R3 primer for HCV
<400> 14
   catggtgcac ggtctacg 18
<210> 15
   <211> 15
   <212> DNA
   <213> Artificial
<220>
   <223> Loop R primer for HCV
<400> 15
   ttgggttgcg aaagg 15
<210> 16
   <211> 44
   <212> DNA
   <213> Artificial
<220>
   <223> FIP primer for HBV
<400> 16
   gataaaacgc cgcagacaca tccttccaac ctcttgtcct ccaa 44
<210> 17
   <211> 46
   <212> DNA
   <213> Artificial
<220>
   <223> RIP primer for HBV
<400> 17
   cctgctgcta tgcctcatct tctttgacaa acgggcaaca tacctt 46
<210> 18
   <211> 20
   <212> DNA
   <213> Artificial
<220>
   <223> F3 primer for HBV
<400> 18
   caaaattcgc agtccccaac 20
<210> 19
   <211> 19
   <212> DNA
   <213> Artificial
<220>
   <223> R3 primer for HBV
<400> 19
   ggtggttgat gttcctgga 19
<210> 20
   <211> 20
   <212> DNA
   <213> Artificial
<220>
   <223> Loop R primer for HBV
<400> 20
   gttggttctt ctggactacc 20
<210> 21
   <211> 20
   <212> DNA
   <213> Artificial
<220>
   <223> probe for HBV
<400> 21
   cagcgatagc caggacaaag 20

## Description

### Technical Field

The present invention relates to a nucleic acid amplification method for use on a porous support, and it also relates to a total system for nucleic acid extraction, amplification, and detection using the same.

### Background Art

Recently, the general demand for DNA testing has been increasing. In order to meet such demand, the development of a simple and compact system whereby nucleic acid extraction from a specimen, followed by amplification and detection of such amplification, can consistently be carried out has been awaited.

In terms of biochemical analysis for enzymes and blood components, a simple solid phase test system based on dry chemistry has been developed (see, for example, Patent Document 1). In such system, reagents that are necessary for a reaction are provided in a matrix in a dry state, so that the reaction takes place with the use of the moisture of an added specimen, which serves as a solvent. In the case of a test system based on dry chemistry, a target component can readily be measured only by spotting a minute amount of a specimen onto a solid phase. Thus, since such system has practical use, it is widely applied in the field of biochemistry.

However, there are some hurdles to be overcome in order to apply the aforementioned dry chemistry system to nucleic acid tests. Firstly, nucleic acid extraction, amplification, and detection have conventionally been carried out via different systems. Thus, it is difficult to integrate such systems into a single system. In particular, a complicated high-temperature cycle is necessary for nucleic acid amplification by PCR or the like. Moreover, reagents such as a template nucleic acid, an enzyme, a substrate, and a primer must be subjected to a reaction system with high degrees of freedom. Therefore, in general, a solid-phase reaction system is not appropriate for nucleic acid tests.

Patent Document 6 discloses microcapsules encapsulating a nucleic acid amplification reaction mixture.

Meanwhile, there are reports regarding methods wherein nucleic acids are allowed to adsorb to a filter, which is made up of nonwoven fabric, filter paper, hydroxyapatite, or the like, so as to be amplified by the LAMP method (see Patent Documents 2 and 3). In accordance with such methods, nucleic acid amplification is carried out by allowing nucleic acids to adsorb to a solid support and adding a LAMP reaction solution thereto.

The LAMP method is a nucleic acid amplification method developed by the inventors of the present invention, and it does not require the complicated temperature control that is considered to be essential for PCR. Thus, a long-chain amplification product having a particular inverted repeat structure (consisting of alternately inverted repeats on the same strand) can be synthesized with high amplification efficiency (see Non-Patent Document 1 and Patent Document 4). The inventors of the present invention have reported a method for detecting nucleic acid amplification on a hydrophilic substrate, comprising allowing a nucleic acid precipitant to bind to a LAMP amplification product (see Patent Document 5). However, the purpose of such method is to solve problems in terms of B/F separation upon hybridization assay. Thus, such method is not intended to establish a system whereby nucleic acid extraction, amplification, and detection are consistently carried out on a solid phase.
[Patent Document 1] JP Patent Publication (Kokai) No. 5-80049 A (1993)
[Patent Document 2] JP Patent Publication (Kokai) No. 2004-201607 A
[Patent Document 3] WO03/6650
[Patent Document 4] WO00/28082
[Patent Document 5] JP Patent Publication (Kokai) No. 2004-141159 A
[Patent Document 6] WO 03/078659 A3
[Non-Patent Document 1] Tsugunori Notomi et al., "Loop-mediated isothermal amplification of DNA," Nucleic Acids Res., vol. 28, No.12: e63, (2000)

### Disclosure of the Invention

In past studies, the inventors of the present invention have established a technique wherein nucleic acid extraction from a specimen is carried out on a solid phase such that a target nucleic acid is introduced onto a solid phase and a method wherein a LAMP product-polyethylenimine (PEI) complex is supplied to a solid phase such that sequence-specific detection is carried out. Thus, if it is possible for a LAMP reaction to take place on a solid phase, all of the steps of extraction, amplification, and detection can be carried out on a solid phase, and thus a total system for nucleic acid analysis can be constructed.

Specifically, it is an objective of the present invention to construct a total system for nucleic acid analysis whereby the steps of extracting, amplifying, and detecting a nucleic acid can all be performed on a solid support.

The inventors of the present invention have confirmed that LAMP reaction takes place by heating a small piece of a filter paper, to which a minute amount of a LAMP reaction solution has been applied, to a predetermined temperature. Further, they have confirmed that LAMP reaction takes place by separating a LAMP reaction solution into several components, supplying the components to a plurality of pieces of filter paper, and overlapping the pieces with each other. Based on these results, they carried out model experiments relating to a total system combining a solid phase extraction method, a solid phase LAMP reaction, and solid phase PEI detection. Accordingly, they succeeded in detecting a target nucleic acid. This has led to the completion of the present invention.

Specifically, the present invention relates to a nucleic acid analysis method according to claim 1 comprising adding a nucleic-acid-containing specimen to a porous support that preliminarily contains reagents for nucleic acid amplification, and then carrying out amplification of a target nucleic acid.

In accordance with the method described above, it is possible to consistently perform nucleic acid extraction from a nucleic-acid-containing specimen, a nucleic acid amplification reaction for a target nucleic acid, and detection of the nucleic acid amplification reaction or the product thereof on the aforementioned porous support.

In such case, at a preliminary stage, the porous support may further contain a reagent for nucleic acid extraction and/or a reagent for nucleic acid detection.

In one embodiment, the method of the present invention is a nucleic acid analysis method according to claim 3, comprising the following steps carried out on a porous support composed of two or more layers:
1) extracting a nucleic acid using a porous support layer containing a reagent for nucleic acid extraction;
2) performing a nucleic acid amplification reaction for a target nucleic acid using a porous support layer containing a reagent for nucleic acid amplification; and
3) hybridizing a nucleic acid probe with the product of a nucleic acid amplification reaction, allowing a nucleic acid precipitant to act on the hybrid that has been produced so as to form an aggregate, and detecting the target nucleic acid in a specimen with the use of the obtained aggregate.

In accordance with the method described above, at a preliminary stage, the porous support may contain a nucleic acid precipitant and/or a nucleic acid probe.

In accordance with the method of the present invention, the porous support is composed of two or more porous supports containing, at a preliminary stage, a reagent for nucleic acid amplification, a reagent for nucleic acid extraction, and/or a reagent for nucleic acid detection.

The reagent for nucleic acid amplification are separately retained within two or more porous supports, with such porous supports then being allowed to come into contact with each other when used upon amplification. For instance, a primer and the other reagent for nucleic acid amplification may each be retained separately within different porous supports. Also, a polymerase and the other reagent for nucleic acid amplification may each be retained separately within different porous supports. Alternatively, the polymerase, the primer, and the other reagent for nucleic acid amplification may each be retained separately within different porous supports. In the aforementioned methods, the polymerase in the dry state is preferably retained within the porous supports.

In accordance with the method of the present invention, the nucleic acid amplification reaction is preferably a LAMP reaction.

In accordance with the method of the present invention, the porous support that can be used is made of at least one material selected from the group consisting of filter paper, nylon membrane, cellulose ester, cellulose, nonwoven fabric, woven fabric, cotton, polyurethane, and sintered plastics.

In addition, the nucleic acid precipitant that can be used is at least one member selected from the group consisting of hydroxyapatite, polyethylenimine, protamine sulfate, poly-L-lysine, and diethylaminoethyl dextran.

In accordance with the present invention, a nucleic acid analysis system for consistently performing nucleic acid extraction, amplification, and detection, such system comprising at least one porous support containing, at a preliminary stage, a reagent for nucleic acid amplification, a reagent for nucleic acid extraction, and/or a reagent for nucleic acid detection is provided.

Examples of reagents contained in the porous support include, but are not limited to:
i) a primer or a labeled primer;
ii) a nucleic acid probe or a labeled probe;
iii) a nucleic acid precipitant;
iv) DNA polymerase; and
v) a nucleotide or a labeled nucleotide that serves as a substrate of a DNA polymerase.

In accordance with the present invention, a solid-phase system capable of consistently performing nucleic acid extraction, amplification, and detection is provided. The system of the present invention can realize simple DNA analysis or DNA tests in clinical practice or laboratories, as examples of applications of nucleic acid analysis to dry chemistry.

### Brief Description of the Drawings

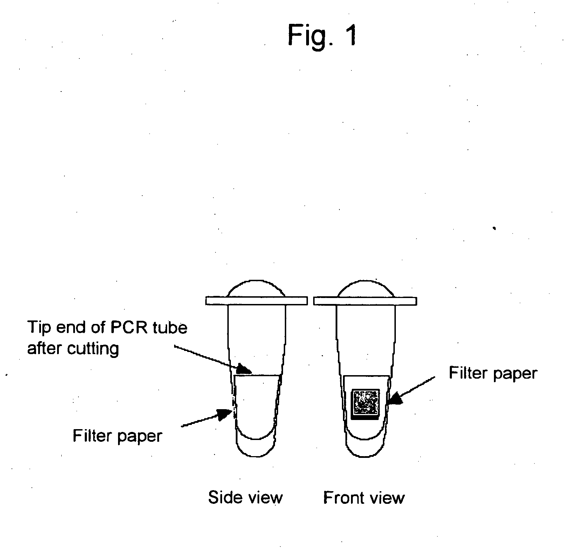

Fig. 1 shows a schematic view of a LAMP reaction on filter paper.

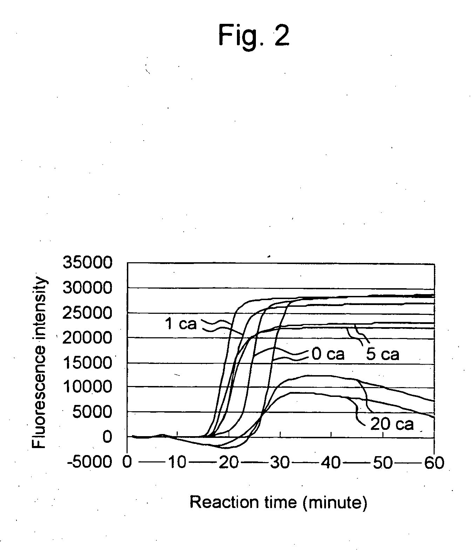

Fig. 2 shows the results of a LAMP reaction on filter paper (CK20: calcein fluorescence detection).

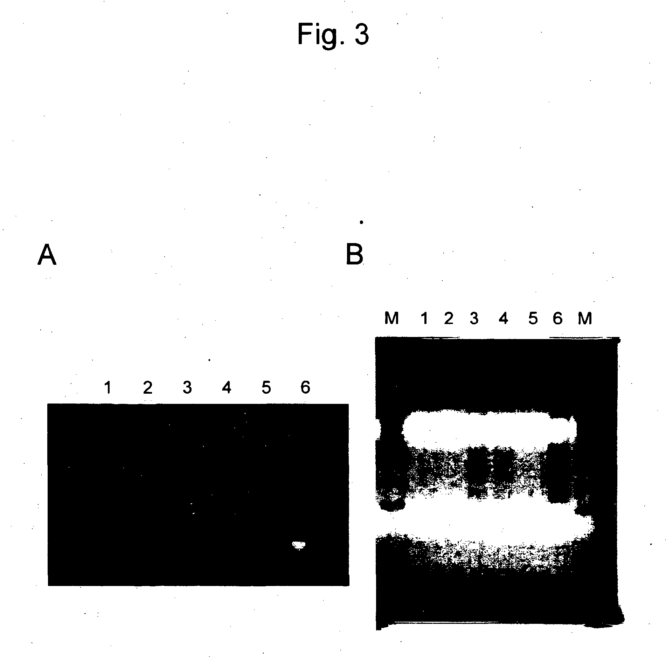

Fig. 3(A) and 3(B) show the results of a LAMP reaction on filter paper (PSA: calcein fluorescence detection). Fig. 3(A) shows the results of visual detection of calcein fluorescence on filter paper (1 and 2: filter paper method (negative); 3 and 4: filter paper method (positive); 5: solution method (negative); and 6: solution method (positive)). Fig. 3(B) shows the results of 3% agarose electrophoresis analysis of an amplification reaction solution (1 and 2: filter paper method (negative); 3 and 4: filter paper method (positive); 5: solution method (negative); and 6: solution method (positive)).

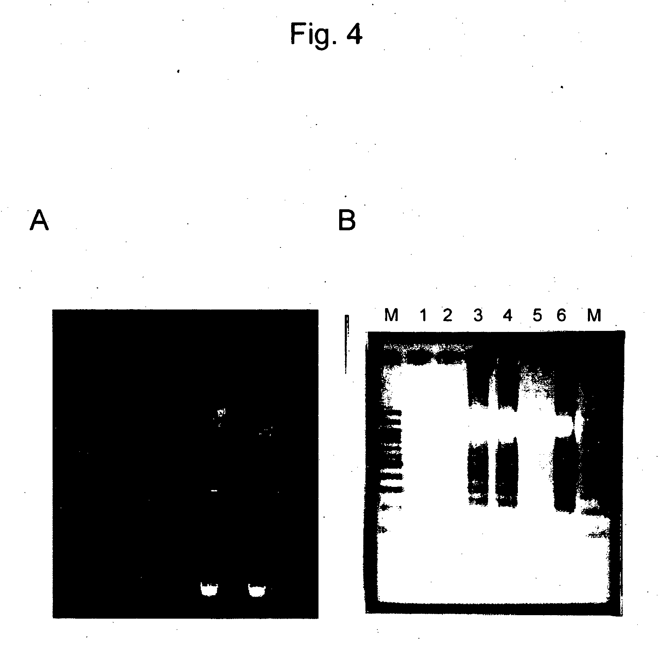

Fig. 4(A) and 4(B) show the results of a LAMP reaction on filter paper based on a combination of a two-piece method and a Bst filter paper method (PSA calcein fluorescence detection). Fig. 4(A) shows the results of visual detection of calcein fluorescence on filter paper (upper column: filter paper method; lower column: solution method). In the upper and lower columns, the two bands at the furthest left are derived from pieces of Bst-free filter paper, which were overlapped with each other, and the two bands at the furthest right are derived from pieces of Bst-containing filter paper, which were overlapped with each other. Fig. 4(B) shows analysis results of 3% agarose electrophoresis using an amplification reaction solution (1 and 2: filter paper method (negative); 3 and 4: filter paper method (positive); 5: solution method (negative); and 6: solution method (positive)).

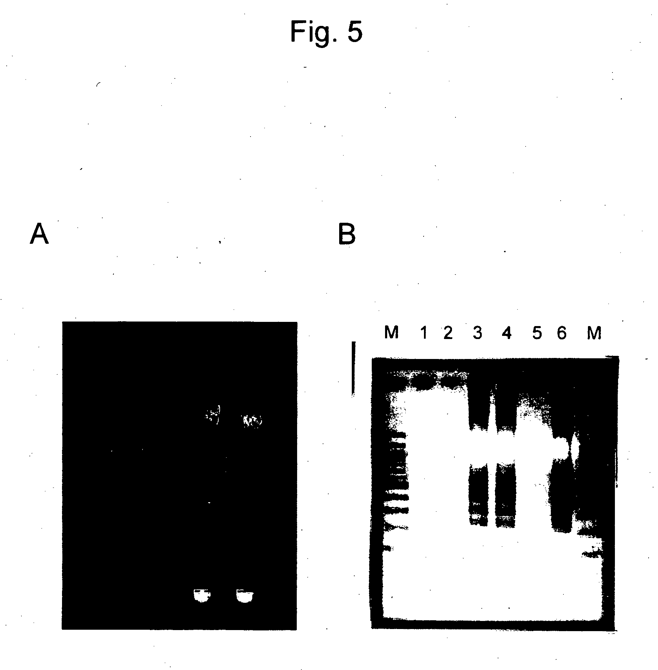

Fig. 5(A) and 5(B) show the results of a LAMP reaction on filter paper based on a combination of a two-piece method and a primer filter paper method (PSA calcein fluorescence detection). Fig. 5(A) shows the results of visual detection of calcein fluorescence on filter paper (upper column: filter paper method; lower column: solution method). In the upper and lower columns, the two bands at the furthest left are derived from pieces of primer-free filter paper, which were overlapped with each other (negative), and the two bands at the furthest right are derived from pieces of primer-containing filter paper, which were overlapped with each other (positive). Fig. 5(B) shows analysis results of 3% agarose electrophoresis using an amplification reaction solution (1 and 2: filter paper method (negative); 3 and 4: filter paper method (positive); 5: solution method (negative); and 6: solution method (positive)).

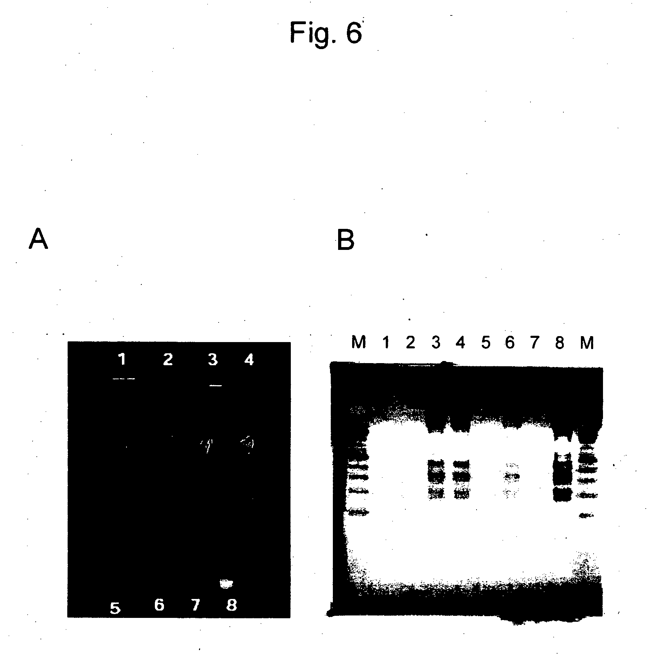

Fig. 6(A) and 6(B) show the results of a LAMP reaction on filter paper based on a three-piece method (HCV transcription RNA calcein fluorescence detection). Fig. 6(A) shows the results of visual detection of calcein fluorescence on filter paper (1 and 2: three-piece filter paper method (negative); 3 and 4: three-piece filter paper method (positive); 5: one-piece filter paper method (negative); 6: one-piece filter paper method (positive); 7: solution method (negative); and 8: solution method (positive)). Fig. 6(B) shows analysis results of 3% agarose electrophoresis using an amplification reaction solution (1 and 2: three-piece filter paper method (negative); 3 and 4: three-piece filter paper method (positive); 5: one-piece filter paper method (negative); 6: one-piece filter paper method (positive); 7 solution method (negative); and 8: solution method (positive)).

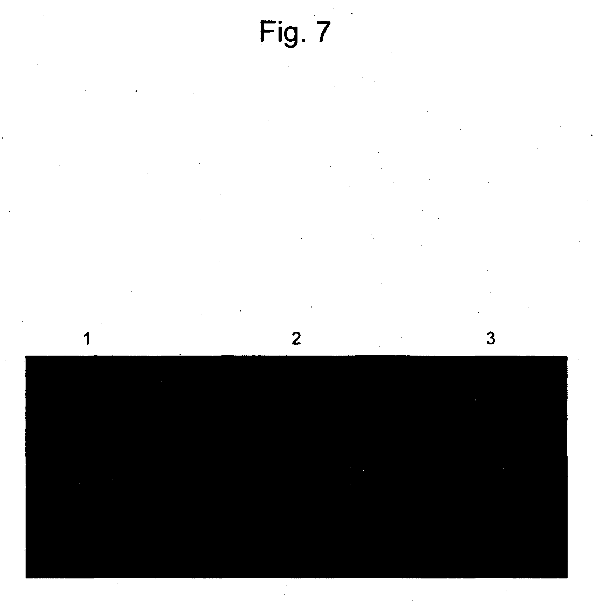

Fig. 7 shows the results of extraction, amplification, and detection that had been carried out on filter paper (1: results of HBV specimen amplification; 2: results of HCV specimen amplification; 3: negative control (not containing an HBV target nucleic acid)).

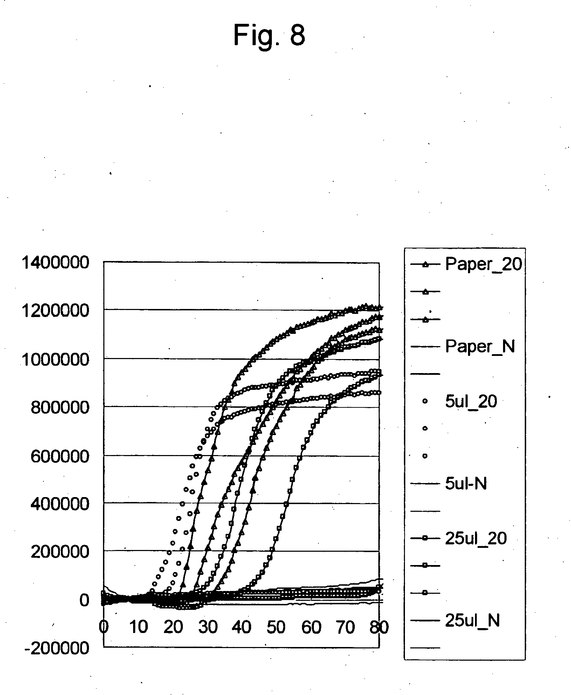

Fig. 8 shows the results of HBV specimen amplification based on a three-piece filter paper method, a 5 µl solution reaction, and a 25 µl-general-scale reaction. In the figure, the three lines with symbol "Δ" (Paper_20) indicate the measurement results of a three-piece filter paper method, the three lines with symbol "O " indicate the measurement results of a 5 µl (5 µl_20) solution reaction, and the three lines with symbol "□" (25 µl_20) indicate the measurement results of a 25 µl general scale reaction. Also, the two lines without symbols (for Paper_N, 5 µl_N, and 25 µl_N) indicate the measurement results for controls.

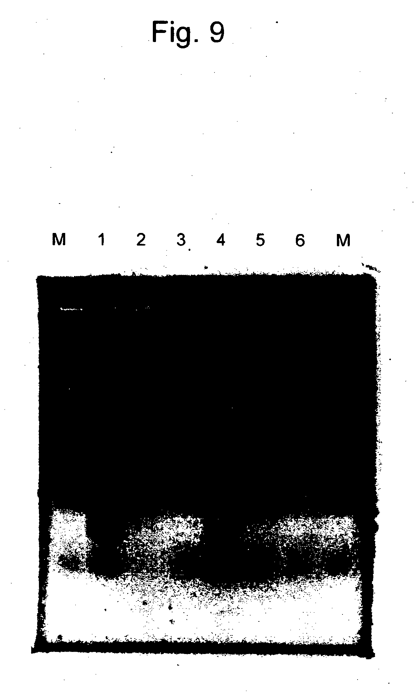

Fig. 9 shows the results of electrophoresis analysis based on a PCR reaction on filter paper (PSA) with the use of a one-piece method (M: 100-bp ladder; 1: filter paper method (negative); 2: template DNA 10⁶ copies/tube (filter paper method (positive)); 3: template DNA 10⁸ copies/tube (filter paper method (positive)); 4: solution method (negative); 5: template DNA 106 copies/tube (solution method (positive)); and 6: template DNA 10⁸ copies/tube (solution method (positive))).

This description includes part or all of the contents as disclosed in the description of Japanese Patent Application No. 2004-252767, which is a priority document of the present application. ,

### Best Mode for Carrying Out the Invention

### 1. Definition of terms

Target nucleic acid: the term "target nucleic acid" in this description indicates a nucleotide sequence or nucleic acid molecule that should be detected.

Porous support: the term "porous support" in this description indicates a porous hydrophilic solid support. Such support is made up of filter paper, nylon membrane, cellulose ester, or the like.

Specimen: the term "specimen" in this description includes any type of nucleic-acid-containing sample, such as a sample of blood or peripheral white blood cells, or nucleic acids extracted from such sample.

Nucleic acid amplification reaction: the term "nucleic acid amplification reaction" in this description includes any known nucleic acid amplification reaction related to a PCR method, an ICAN method, an SDA method, an NASBA method, or a LAMP method.

Reagent for nucleic acid amplification: the term "reagent for nucleic acid amplification" in this description includes any type of reagent that is necessary for nucleic acid amplification reactions and contains DNA polymerases, nucleotide substrates, primers, probes, or the like.

Reagent for nucleic acid extraction: the term "reagent for nucleic acid extraction" in this description includes any type of reagent that is necessary for nucleic acid extraction.

Reagent for nucleic acid detection: the term "reagent for nucleic acid detection" in this description includes any type of reagent that is necessary for nucleic acid extraction, such as luminescent reagent, fluorescent reagent, intercalator, or nucleic acid precipitant.

Primer: the term "primer" in this description indicates an oligonucleotide or a labeled oligonucleotide that is specifically hybridized to a target nucleic acid for amplification.

Nucleic acid probe: the term "nucleic acid probe" in this description indicates a nucleic acid fragment that is specifically hybridized to a target nucleic acid for detection of the sequence of the target nucleic acid. In addition, as described below, a nucleic acid probe may be a peptide nucleic acid or a locked nucleic acid.

Nucleic acid precipitant: the term "nucleic acid precipitant" in this description indicates an agent that adsorbs to a nucleic acid such as polyethylenimine so as to form an aggregate.

In addition, specific examples of the use of each above term will be described below and in Examples in greater detail.

### 2. Nucleic acid extraction on a porous support

Nucleic acid extraction on a porous support can be carried out in accordance with a method described in Example 3 below. For instance, alcohols (e.g., ethanol and isopropanol) are added to a specimen in the coexistence of a protein denaturant, resulting in nucleic acid precipitation such that the precipitant is allowed to be carried on a porous support.

### 3. Nucleic acid amplification on a porous support

Nucleic acids extracted on a porous support are directly subjected to a nucleic acid amplification reaction. A nucleic acid amplification reaction may be carried out in accordance with any known method for nucleic acid amplification such as a PCR method, an ICAN method, an SDA method, an NASBA method, or a LAMP method.

Particularly, a nucleic acid amplification reaction used in the present invention is preferably a LAMP reaction. The LAMP (loop-mediated isothermal amplification) method is a nucleic acid amplification method that has been developed by the inventors of the present invention. In accordance with the method, DNA or RNA can be rapidly amplified at a low cost under isothermal conditions (approximately 65°C) with the use of two, four, or six types of specific primers (an inner primer pair, an inner primer pair with an outer primer pair, or an inner primer pair with an outer primer pair and a loop primer pair), polymerase with strand displacement activity, and nucleotide that serves as a substrate. The outline of the LAMP method is described in detail in the reference: Notomi, T et al.: Nucleic Acids Res. 28(12): e63 (2000); the patent publication: WO00/28082; and on the web site of Eiken Chemical Co., Ltd. (http://www.eiken.co.jp/).

In accordance with the LAMP method, an elongation reaction and an amplification reaction simultaneously progress at a plurality of sites on the same strand of an amplification product. Thus, DNA amplification is superexponentially achieved under isothermal conditions so that a long-chain amplification product having a particular inverted repeat structure (structure consisting of alternately inverted repeats on the same strand) can be synthesized with high amplification efficiency.

In the LAMP method, specific primers that are referred to as an inner primer, an outer primer, and a loop primer are used.

An inner primer is essential for the LAMP method. When an arbitrary sequence X2c that exists on the 3' side and an arbitrary sequence X1c on the 5' side relative to X2c are selected from a strand of each template DNA, an inner primer comprises a sequence X2 (complementary to X2c above) and a sequence X1c (identical to X1c above) from the 3' side to the 5' side in that order. (The primer has a structure of X1c+X2.)

The term "outer primer" indicates two types of primers (each complementary to a different member of a double strand), each having a sequence complementary to an arbitrary sequence X3c on the ouside relative to an inner primer (i.e., on the 3' side of a template) and capable of annealing to X3c.

When alternately inverted sequences, which are generated on the same strand of an amplification product that has been obtained by the LAMP method, anneal to each other so as to form a loop, a primer that is referred to as a "loop primer" comprises a base sequence on the 3' end, such sequence being complementary to a sequence of the loop. The outer primer and the loop primer described above are not essential for the LAMP method. However, with the use of these primers, an amplification reaction can progress with improved efficiency.

A LAMP reaction is carried out at temperatures of, for example, 50°C to 75°C and preferably of 55°C to 70°C for 1 minute to 10 hours and preferably for 5 minutes to 4 hours. At such temperatures, an inner primer can form a stable base-pair binding with a sequence complementary thereto on the template nucleic acid, and a polymerase with strand displacement activity can maintain enzyme activity.

In addition, the LAMP reaction is preferably carried out in the coexistence of a buffer agent that achieves a preferable pH for enzymatic reaction, salts that are necessary for the maintenance of enzymatic catalytic activity and annealing, and an enzyme protectant. Further, the reaction is carried out with the use of a melting temperature regulator (Tm) and the like according to need. Examples of such buffer agent to be used include Tris-HCl having a neutral-weak alkaline buffering action. pH may be adjusted according to the DNA polymerase to be used. Examples of salts that may be adequately added for the maintenance of enzyme activity and the adjustment of DNA melting temperature (Tm) include KCI, NaCl, and (NH)₂SO₄. Examples of enzyme protectants used include bovine serum albumin and saccharides. In addition, examples of a melting temperature regulator (Tm) that can generally be used include betaine, proline, dimethyl sulfoxide, and formamide.

An inner primer or a nucleotide substrate may be labeled with an adequate labeling substance. Examples of labeling substances include fluorescent dyes (e.g., FITC and ROC), enzymes, proteins, radioisotopes, chemiluminescent substances (e.g., DNP), biotin, and DIG (digoxigenin).

### 4. Target nucleic acid detection on a porous support

An amplified target nucleic acid can be readily detected on a porous support in accordance with a known method with the use of a labeled nucleic acid probe, a fluorescent reagent, or the like. A method using a nucleic acid probe and a nucleic acid precipitant is particularly preferable because an amplification product can be visually confirmed in a convenient manner.

### (1) Nucleic acid probe

A nucleic acid probe used for detection is an oligonucleotide probe having a sequence complementary to at least one part of a target nucleic acid. The chain length thereof is generally approximately 5 to 50 bases and preferably approximately 10 to 30 bases.

In particular, when a nucleic acid precipitant is used, it is particularly preferable that such probe have a relatively short chain. A nucleic acid precipitant has a property of easily adsorbing to a long-chain nucleic acid. Thus, in order to achieve good B/F separation while preventing a precipitant from adsorbing to a released probe, it is generally necessary for a nucleic acid probe to have a relatively short chain. However, regardless of the probe length, good B/F separation can be achieved by adjusting reaction conditions such as by adjusting the temperature of a nucleic acid precipitant reaction to be close to the Tm of a probe.

The nucleic acid probe described above may be labeled with an adequate labeling substance. Examples of labeling substances include fluorescent dyes (e.g., FITC and ROC), enzymes, proteins, radioisotopes, chemiluminescent substances (e.g., DNP), biotin, and DIG (digoxigenin). In addition, such nucleic acid probe may be either a peptide nucleic acid (Nielsen, P. E. et al., Science 254, 1497-1500 (1991)) or a locked nucleic acid (WO99/14226 and JP Patent Publication (Kohyo) 2002-521310 A).

### (2) Nucleic acid precipitant

A nucleic acid precipitant used in the present invention is not particularly limited as long as it adsorbs to a nucleic acid so as to form an aggregate. However, a precipitant that selectively binds to a long-chain nucleic acid rather than a short-chain nucleic acid is preferable. Specifically, examples of known nucleic acid precipitants include surfactants, dihydroxybenzene, sodium dodecyl sulfate, diisobutyl sodium sulfosuccinate, tetradecyl sodium sulfate, dihydroxybenzene, Sarkosyl, alkali metal salts containing SO₄, PO₄, Cl, or HCOO, and ammonium salts. In accordance with the present invention, preferred examples thereof are hydroxyapatite, polyethylenimine, protamine sulfate, poly-L-lysine, and diethylaminoethyl dextran (DEAE dextran). A particularly preferred example of polyethylenimine has a degree of polymerization of approximately 40 or less.

The concentration and the amount of a nucleic acid precipitant to be used are determined depending on properties of such precipitant. For instance, in the case of polyethylenimine having a degree of polymerization of 14, a preferred concentration is approximately 0.1 to 2 M and the preferred amount added is approximately 4 to 200 µg per each 25 µl of a reaction solution.

The pH of a reaction solution that is appropriate for a nucleic acid precipitant is determined depending on the properties of such precipitant. However, in general, the pH is preferably 6 to 10. This is because a probe is unlikely to be hybridized to a nucleic acid when the pH of a reaction solution is too high. On the other hand, nucleic acid protonation results in the deterioration in B/F separation when the pH of a reaction solution is too low.

In addition, the ionic strength of a reaction solution upon the addition of a nucleic acid precipitant is also determined depending on the properties of such precipitant. However, in general, the ionic strength is preferably 0.15 mol/l or less. When the ionic strength is too high, the electrostatic interaction between a precipitant and a nucleic acid is inhibited, resulting in insufficient formation of an aggregate.

Further, the temperature upon the addition of a nucleic acid precipitant is preferably approximately 20°C to 70°C. Also, the temperature may be the same as the constant temperature upon nucleic acid amplification.

### (3) Detection using a nucleic acid precipitant

The nucleic acid probe and the nucleic acid precipitant described above may be added to a porous support subjected to amplification, or they may be spotted onto an adequate site of a porous support at a preliminary stage. When a nucleic acid probe is added to a porous support at a preliminary stage, followed by an amplification reaction, the amplification step and the hybridization step described above take place almost simultaneously. Conditions for hybridization of an amplification product with a nucleic acid probe are not particularly limited. However, in general, hybridization may be carried out at 50°C to 70°C for 5 minutes.

An aggregate composed of an amplification nucleic acid, a nucleic acid probe, and a nucleic acid precipitant clearly differs from a released nucleic acid probe in terms of mobility on a solid phase. Thus, they can be readily separated from each other.

An aggregate can be readily detected by labeling a nucleic acid probe or an amplification product. Detection involves qualitative detection and quantitative detection. For instance, qualitative detection of the presence or absence of a target sequence or the like can readily be carried out by visual confirmation of an aggregate. Meanwhile, quantitative detection can be carried out based on signal intensity measurement using a commercially available apparatus such as a "Polarion" fluorescence plate reader (TECAN).

### 5. Nucleic acid analysis system

In accordance with the present invention, a nucleic acid analysis system for consistently performing nucleic acid extraction, amplification, and detection is provided, which comprises at least one porous support containing, at a preliminary stage, a reagent for nucleic acid amplification, a reagent for nucleic acid extraction, and/or a reagent for nucleic acid detection.

Examples of a reagent contained in a porous support include, but are not particularly limited to:
i) a primer or a labeled primer;
ii) a nucleic acid probe or a labeled nucleic acid probe;
iii) a nucleic acid precipitant;
iv) DNA polymerase; and
v) a nucleotide or a labeled nucleotide that serves as a substrate of DNA polymerase.

In the case of LAMP amplification, it is desired that the aforementioned primer further contain an outer primer and/or a loop primer or a labeled loop primer as well as an inner primer.

In addition, as described above, a nucleic acid precipitant is preferably at least one member selected from the group consisting of hydroxyapatite, polyethylenimine, protamine sulfate, poly-L-lysine, and diethylaminoethyl dextran.

Further, the system of the present invention may be provided as a kit comprising a melting temperature regulator (e.g., betaine and trimethylamine N-oxide), a buffer with which preferred conditions for enzymatic reaction are obtained, and/or other reagents that are necessary for detection of a synthetic reaction product according to need.

The system of the present invention is structured in a manner such that a reagent for nucleic acid amplification is separately retained within two or more porous supports that are allowed to come into contact with each other upon use (or amplification). Examples of such system include a system in which a primer and the other reagents for nucleic acid amplification are separately retained within different porous supports, a system in which polymerase and the other reagents for nucleic acid amplification are separately retained within different porous supports, and a system in which polymerase, a primer, and the other reagents for nucleic acid amplification are separately retained within different porous supports. In such systems, polymerase in the dry state is preferably retained within a porous support, in view of the maintenance of stability.

### Examples

The present invention is hereafter described in greater detail with reference to the following Examples, although the technical scope of the present invention is not limited thereto.

In addition, a LAMP reaction solution and testing materials described below were used in the Examples, unless otherwise specified.
(1) LAMP reaction solution

**[Table 1]**

| Basic composition of LAMP reaction solution | |
|---|---|
| Tris-HCl (pH 8.8) | 20 mM |
| KCl | 10 mM |
| (NH₄)₂SO₄ | 10 mM |
| MgSO₄ | 8 mM |
| Tween20 | 0.1% |
| Betain | 0.8 M [0.6 M] |
| dNTPs | 5.6 mM [7.6 mM] |
| Inner primer | 3.2 µM |
| Outer primer | 0.8 µM |
| Loop primer | 1.6 µM |
| Bst polymerase | 8 U/tube |
| BSA | 2% |
| (enzvme mix 1 µl/tube) | |

| | |
|---|---|
| []: for an HCV amplification system ( ): for a CK20 or HCV amplification system | |

(2) Target nucleic acids, primers, and labeled probes
a) PSA (PSA cDNA GenBank ID: M26663 (cloned into pBR322))
   FIP TGTTCCTGATGCAGTGGGCAGCTTTAGTCTGCGGCGGTGTTCTG (SEQ ID NO: 1)
   RIP TGCTGGGTCGGCACAGCCTGAAGCTGACCTGAAATACCTGGCCTG (SEQ ID NO: 2)
   F3 TGCTTGTGGCCTCTCGTG (SEQ ID NO: 3)
   R3 GGGTGTGTGAAGCTGTG (SEQ ID NO: 4)
b) CK20 (GenBank ID: BC031559)
   FIP: CAATTTGCAGGACACACCGAGATTGAAGAGCTGCGAAGTC (SEQ ID NO: 5)
   BIP: CTGCTGAGGACTTCAGACTGACTTGGAGATCAGCTTCCAC (SEQ ID NO: 6)
   F3: CGA CTACAGTGCATATTACAGAC (SEQ ID NO: 7)
   B3: GTAGGGTTAGGTCATCAAAGAC (SEQ ID NO: 8)
   LpF: GCAGTTGAGCATCCTTAATCT (SEQ ID NO: 9)
   LpB: GACTGAGAGAGGAATACGTC (SEQ ID NO: 10)
c) HCV specimen (pack specimen of "Nisseki" plasma (Akita): ABC No. 62: approximately 2300 KIU/ml; 1.15×10⁴ copies/µl)
   FIP: GGTTKATCCAAGAAAGGACCCAGTCGCCATAGTGGTCTGCGGA (SEQ ID NO: 11)
   BIP: CCGCAAGACTGCTAGCCGAGGCAAGCACCCTATCAGGC (SEQ ID NO: 12)
   F3: GGCGTTAGTATGAGTGTCGTAC (SEQ ID NO: 13)
   B3: CATGGTGCACGGTCTACG (SEQ ID NO: 14)
   Loop R: TTGGGTTGCGAAAGG (SEQ ID NO: 15)
d) HBV specimen (pack specimen of "Nisseki" plasma No. 79: 63100 copies/ml)
   FIP: GATAAAACGCCGCAGACACATCCTTCCAACCTCTTGTCCTCCAA (SEQ ID NO: 16)
   BIP: CCTGCTGCTATGCCTCATCTTCTTTGACAAACGGGCAACATACCTT (SEQ ID NO: 17)
   F3: CAAAATTCGCAGTCCCCAAC (SEQ ID NO: 18)
   B3: GGTGGTTGATGTTCCTGGA (SEQ ID NO: 19)
   Loop R: GTTGGTTCTTCTGGACTACC (SEQ ID NO: 20)
   Labeled HBV probe (3'ROX-labeled): CAGCGATAGCCAGGACAAAG (SEQ ID NO: 21)

(3) Filter paper
As a filter paper material, qualitative filter paper No. 1 (produced by ToyoRoshi Kaisha, Ltd.) cut into squares 3 mm on each side or circles 4 mm in diameter (hereafter referred to as "piece(s) of filter paper") were used.

### [Example 1] Experiment of adding filter paper to a LAMP reaction solution

First, effects of the addition of filter paper (cellulose) during the general scale of LAMP reaction were examined in a system using CK20 in accordance with the following method.

### 1. Experimental method

50 µl of a LAMP reaction solution (table 1) was added to a 0.2-ml PCR tube. A predetermined number of pieces of filter paper were added thereto. CK20 was used as a target nucleic acid. LAMP amplification was performed by heating at 63°C for 60 minutes with the use of a thermal cycler. Calcein fluorescence (FAM Dye layer) was measured using ABI-7700 (Applied Biosystems) so that the presence or absence of amplification was confirmed.

### 2. Experimental results

Fig. 2 shows the results. As shown in fig. 2, even after the addition of 20 pieces of filter paper, the amplification reaction was confirmed to take place. Since each piece of filter paper (3-mm square) was able to retain up to 3 µl of liquid, 20 pieces of filter paper were able to retain not less than 50 µl of liquid. That is, it has been confirmed that the LAMP reaction takes place not only when adding filter paper to a reaction solution (with an excessive amount of a reaction solution) but also when supplying a reaction solution to filter paper (with an excessive amount of filter paper).

### [Example 2] LAMP reaction on filter paper

### 1. Experimental method

A reaction was performed with the addition of 0 to 20 pieces of filter paper (3-mm squares). The LAMP reaction was performed by heating a PCR tube to a given temperature using a thermal cycler. The presence or absence of amplification was confirmed by adding calcein (Dojindo Laboratories) thereto so as to detect fluorescence (ABI-7700 (with the use of FAM Dye layer), Applied Biosystems). Next, 10 µl of Loading Dye (6 x) (produced by Promega) was added to the pieces of filter paper. The pieces of filter paper were allowed to stand for 5 minutes, followed by 3% agarose electrophoresis. Accordingly, LAMP amplification of the target nucleic acid was confirmed. The LAMP reaction on filter paper was performed in accordance with the different methods described below.

### 1. 1 One-piece method

3 µl of a LAMP reaction solution containing all components listed in table 1 was supplied to a single piece of filter paper, followed by the LAMP reaction.

### 1. 2 Two-piece method

In a case in which primers, enzymes, substrates, and magnesium ions exist in a single solution, a long period of preservation might cause a nonspecific reaction that would result in the formation of a primer dimer or the like. Thus, it was examined in a system using PSA whether or not it would be possible to induce a LAMP reaction by supplying primers or enzymes to a piece of filter paper, which differs from other pieces used for the other reagents, and overlapping such pieces with each other when necessary.

### (1) Primer filter paper method

3 µl of a LAMP reaction solution not containing primers alone (RM: 1.67-fold concentration) and 2 µl of a 2.5-fold concentrated primer solution were supplied to separate pieces of filter paper. A LAMP reaction was performed by overlapping the pieces with each other. In addition, the concentration of each component was adjusted in a manner such that a given concentration would be achieved when the two pieces were overlapped with each other, resulting in a total liquid volume of 5 µl.

### (2) Bst filter paper method

A LAMP reaction solution not retaining Bst alone (RM: 1.67-fold concentration) (3 µl) and 4-fold diluted Bst (or Enzyme Mix) stock solution (2 µl) were supplied to separate pieces of filter paper. The reaction was performed by overlapping the pieces with each other. In addition, the concentration of each component was adjusted in a manner such that a predetermined concentration would be achieved when overlapping the two pieces with each other, resulting in a total liquid volume of 5 µl.

### 1. 3 Three-piece method

In order to combine a filter paper reaction method with a filter paper extraction method, a LAMP reaction must be carried out on filter paper on which a target nucleic acid is carried. This means that a filter paper reaction involving 3 pieces of filter paper (filter paper containing a target nucleic acid, RM filter paper, and Bst filter paper (or Enzyme Mix filter paper)) is necessary. Thus, a three-piece method was conducted in a system using HCV in accordance with the following method.

3 µl of a LAMP reaction solution (RM: 1.67-fold concentration) not containing Bst and a target nucleic acid, 1 µl of a 2-fold diluted Bst solution (or Enzyme Mix), and 1 µl of a target nucleic acid solution at an arbitrary concentration were separately supplied to 3 pieces of filter paper. The pieces were overlapped with each other so that a LAMP reaction was performed. In addition, the concentration of each component was adjusted in a manner such that a given concentration would be achieved when overlapping the three pieces with each other, resulting in a total liquid volume of 5 µl.

### 2. Experimental results and discussion

### 2. 1 One-piece method

Fig. 3 shows the results of the LAMP reaction performed on filter paper at 65°C for 60 minutes with the use of PSA (6 x 10⁻²⁰ M) as a target nucleic acid. As shown in Fig. 3, calcein fluorescence was observed exclusively when the target nucleic acid was contained. Thus, LAMP amplification was confirmed. In addition, based on 3% agarose electrophoresis analysis, a ladder pattern in a given size was obtained. Therefore, it has been confirmed that it is possible to perform a LAMP reaction on filter paper.

### 2. 2 Two-piece method

Fig. 4 shows the results of the LAMP reaction performed on filter paper at 65°C for 60 minutes with the use of PSA (6 x 10⁻²⁰ M) as a target nucleic acid in accordance with the primer filter paper method. In addition, Fig. 5 shows the results of the LAMP reaction performed on filter paper at 65°C for 60 minutes with the use of PSA (6 x 10⁻²⁰ M) as a target nucleic acid in accordance with the Bst filter paper method.

Based on figs. 4 and 5, even in a case in which primers and enzymes were supplied to different pieces of filter paper, it was confirmed that a given LAMP reaction took place by overlapping the pieces of filter paper with each other. That is, it has been confirmed that primers and Bst are sufficiently smaller than the pores of filter paper, and thus they can freely migrate between pieces of filter paper.

The objective of this experiment was to confirm the possibility of the migration of a substance (primer or enzyme) between two pieces of filter paper. Thus, a reaction condition for a negative control was the absence of primers or enzymes but not the absence of a target nucleic acid. (One of the two pieces was designated as water spot filter paper.)

It has been known that Bst can be stably formulated on filter paper. Thus, it is considered that a LAMP reagent can be formulated in accordance with the Bst filter paper method. Meanwhile, RM filter paper can be advantageously standardized for any system by supplying primers to a separate piece of filter paper. Thus, such case can be applied to simultaneous detection of a plurality of items or targets.

### 2. 3 Three-piece method

Fig. 6 shows the results of the LAMP reaction performed on filter paper at 63°C for 60 minutes with the use of HCV transcription RNA (6 x 10⁻¹⁸ M) as a target nucleic acid in accordance with the three-piece method. Also, in accordance with the three-piece method, a given level of LAMP amplification was confirmed. Thus, it has been confirmed that it is possible to construct a system in which filter paper that carries target nucleic acids that have been extracted via filter paper is overlapped with two separate pieces of filter paper on which a LAMP reagent has been formulated.

The inventors have confirmed that target nucleic acids pass through filter paper so as not to be carried on filter paper when carrying out a nucleic acid extraction method using filter paper, wherein filtration operations are performed without using effects of isopropanol. This has revealed that sizes of target nucleic acids that have been precipitated (or have aggregated) with isopropanol are larger than the pore sizes of filter paper; however, the sizes of dissolved target nucleic acids are sufficiently smaller than the pore sizes of filter paper. In addition, it has been revealed that there is no specific interaction, such as adsorption, between dissolved target nucleic acids and filter paper (cellulose). Accordingly, it was estimated that dissolved target nucleic acids can freely migrate between pieces of filter paper to some extent, so that solutions contained in three overlapped pieces of filter paper collectively function as a given LAMP reaction solution (i.e., the same solution used in a one-piece method) containing target nucleic acids.

### [Example 3] Model experiment of a total system using filter paper

A model experiment of a total system using filter paper was carried out in accordance with the following protocols.

### 1. Experimental method

(1) A specimen used was a solution of an HCV specimen (pack specimen of "Nisseki" plasma (Akita); ABC No. 62; approximately 2300 KIU/ml; 1. 15 x 104 copies/µl) that had been diluted 1000-fold with phosphate buffer or a solution of an HBV specimen (pack specimen of "Nisseki" plasma No. 79; 63100 copies/ml) that had been diluted 10-fold with normal plasma (No. 39). 100 µl of the specimen was mixed with 300 µl of lysis buffer (containing 68% guanidine thiocyanate, 3%DTT, and 10 mM Tris (pH 8.0)) and the resultant was allowed to stand for 10 minutes. Next, 400 µl of 100% isopropanol was added thereto, followed by filtration through a piece of filter paper (diameter: 4 mm; No. 1) with the use of a syringe.
(2) The piece of filter paper carrying target nucleic acids was washed with 500 µl of 70% ethanol by allowing the ethanol to pass therethrough with the use of a syringe.
(3) The piece of filter paper was allowed to stand for 5 minutes such that ethanol was removed therefrom.
(4) The obtained filter paper was selected to serve as target nucleic acid filter paper and it was overlapped with filter paper containing RM or Bst (or Enzyme Mix) filter paper. They were overlapped with each other and put into a PCR tube, followed by heating at 63°C for 1 hour.
(5) The filter papers subjected to the reaction were overlapped with a piece of filter paper onto which 1.2 µl of a solution of polyethylenimine (PEI: degree of polymerization of 14) containing 0.75 M KCI at a monomer concentration of 0.25 M had been spotted. Then, they were allowed to stand for 1 minute. A solution containing 100 nM ROX-labeled HBV probe (100 µl) was added to these filter papers, followed by hybridization of the probe to a LAMP product-PEI complex. Subsequently, a filter paper to which the LAMP product-PEI complex had been supplied was visually observed on a fluorescent illuminator (302 nm). In addition, an HCV amplification product was used as a negative control (irrelevant LAMP product) upon probe detection. Further, an HBV amplification system not containing target nucleic acids was used as a negative control upon LAMP reaction.

### 2. Experimental results and discussion

Amplification reaction based on the three-piece method and PEI detection using filter paper were sequentially carried out in such order with the use of HBV extracted on filter paper as a target nucleic acid (Fig. 7). As a result, a series of genetic tests (including sequence-specific detection) with the use of a substantial HBV specimen were successfully carried out on filter paper. Specifically, a sample of an amplified HBV specimen (Fig. 7-1) exclusively exhibited red fluorescence of ROX. In the case of the irrelevant LAMP product (HCV specimen: Fig. 7-2) and the negative control (Fig. 7-3), fluorescence derived from filter paper was observed to a slight extent.

Based on the above results, it has been revealed that it is possible to induce a LAMP reaction on a porous solid carrier such as filter paper, as with the case of a LAMP reaction induced in a liquid phase. In addition, it has also been confirmed that a LAMP reaction takes place in a case in which components of a reaction system are supplied to separate pieces of filter paper that are then overlapped with each other. This indicates that it is possible to consistently carry out all the steps of extraction, amplification, and detection on filter paper. If the steps can be combined on a single device, such method can be applied to a simple and inexpensive total system for nucleic acid detection (analysis).

Based on this study, it has been confirmed that, with the use of a total system comprising filter paper as a base carrier, incorporation of even a minute amount of a reagent can be avoided, a solution feeding system in a device can be replaced with a simple carrier migration system, and a reagent can be stabilized.

### [Example 4] Sensitivity of the three-piece method

### 1. Experimental method

In order to evaluate the sensitivity of the three-piece method, amplification and detection of an HBV specimen (20 copies/tube) were carried out by the following three methods based on Examples 1 to 3.
(1) Three-piece filter paper method: 5 µl (RM 3 µl + 1/2 Bst 1 µl + template 1 µl)
(2) solution reaction: 5 µl (RM 3 µl + 1/2 Bst 1 µl + template 1 µl)
(3) general scale reaction: 25 µl

Detection was carried out for samples (3 tubes) using a "FluoDia T70" fluorescence plate reader (Ex: 486 nm and Em: 530 nm, Otsuka Electronics Co., Ltd). In addition, samples not containing a specimen (2 tubes) serving as controls were subjected to amplification and detection in a similar manner.

### 2. Experimental results and discussion

Fig. 8 shows the results. Accordingly, in the case of the solution reaction, amplification was observed in 2 out of 3 tubes. Thus, it was considered that the limit of sensitivity was 20 copies/tube under the conditions of Example 4. Meanwhile, in accordance with the three-piece filter paper method, amplification was observed in 3 out of 3 tubes. Thus, it has been confirmed that the sensitivity of the filter paper of the present invention is equivalent to or higher than the sensitivity of the solution method.

### [Example 5] PCR reaction on filter paper

In accordance with the one-piece method of Example 2, PCR reaction was performed on filter paper using PSA (6 x10⁻²⁰ M) as a target nucleic acid with the addition of 3 µl of a PCR reaction solution per single piece of filter paper. (The amplification region had a size of 178 bp.) Based on the PCR reaction solution composition given in table 2, PCR reaction was performed at 95°C for 30 seconds, at 58°C for 30 seconds, and at 70°C for 30 seconds for 35 cycles with the use of outer primers (SEQ ID NOS: 3 and 4) of PSA LAMP primer as a PCR primer based on TAKARA LA Taq standard protocols.

**[Table 2]**

| PCR reaction solution composition | |
|---|---|
| Primer | 0.5 µM each |
| LA Taq | 2.5 U |
| 10 × LA PCR buffer II | 1 µl |
| 25 mM MgCl₂ | 2.5 mM |
| 2.5 mM each dNTP | 0.4 mM each |
| Betain | 0.5 M |
| BSA | 0.9% |
| Total | 1 0 µl |

Three pieces of filter paper subjected to the reaction were impregnated with 10 µl of a loading dye, followed by electrophoresis. Thus, amplification was confirmed. By way of comparison, standard solution PCR was carried out under similar PCR conditions. The solution PCR was carried out at a scale of 10 µl. All the PCR products were subjected to electrophoresis.

As shown in fig. 9, also in the case of PCR performed on filter paper, a band corresponding to the size of an amplification region was confirmed, as with the case of solution PCR. Thus, it has been confirmed that nucleic acid amplification can be carried out on filter paper, as with the case of the LAMP method even with the use of PCR.

All publications, patents, and patent applications cited herein are incorporated herein by reference in their entirety.

### Industrial Applicability

The present invention relates to a simple nucleic acid detection device for consistently performing nucleic acid extraction, amplification, and detection. Such device can be used for simple DNA analysis or DNA test in clinical practices or laboratories.

### Free Text of Sequence Listing

SEQ ID NO: 1-description of an artificial sequence: FIP primer for PSA
SEQ ID NO: 2-description of an artificial sequence: RIP primer for PSA
SEQ ID NO: 3-description of an artificial sequence: F3 primer for PSA
SEQ ID NO: 4-description of an artificial sequence: R3 primer for PSA
SEQ ID NO: 5-description of an artificial sequence: FIP primer for CK20
SEQ ID NO: 6-description of an artificial sequence: RIP primer for CK20
SEQ ID NO: 7-description of an artificial sequence: F3 primer for CK20
SEQ ID NO: 8-description of an artificial sequence: R3 primer for CK20
SEQ ID NO: 9-description of an artificial sequence: LpF primer for CK20
SEQ ID NO: 10-description of an artificial sequence: LpR primer for CK20
SEQ ID NO: 11-description of an artificial sequence: FIP primer for HCV
SEQ ID NO: 12-description of an artificial sequence: RIP primer for HCV
SEQ ID NO: 13-description of an artificial sequence: F3 primer for HCV
SEQ ID NO: 14-description of an artificial sequence: R3 primer for HCV
SEQ ID NO: 15-description of an artificial sequence: Loop primer for HCV
SEQ ID NO: 16-description of an artificial sequence: FIP primer for HBV
SEQ ID NO: 17-description of an artificial sequence: RIP primer for HBV
SEQ ID NO: 18-description of an artificial sequence: F3 primer for HBV
SEQ ID NO: 19-description of an artificial sequence: R3 primer for HBV
SEQ ID NO: 20-description of an artificial sequence: Loop primer for HBV
SEQ ID NO: 21-description of an artificial sequence: probe for HBV

## Claims

1. A nucleic acid analysis method comprising adding a nucleic acid-containing specimen to a porous support containing a reagent for nucleic acid amplification and allowing a target nucleic acid to be subjected to a nucleic acid amplification reaction, wherein
a) a primer and the other reagents for nucleic acid amplification are separately retained within different porous supports, or
b) a polymerase and the other reagents for nucleic acid amplification are separately retained within different porous supports, or
c) the polymerase, the primer, and the other reagents for nucleic acid amplification are separately retained within different porous supports,
and the porous supports are allowed to come into contact with each other when used upon amplification.

2. The nucleic acid analysis method according to claim 1, comprising consistently performing a nucleic acid extraction from the specimen, an amplification reaction for the target nucleic acid, and detection of the nucleic acid amplification reaction or the product thereof on the porous support.

3. A nucleic acid analysis method, comprising the following steps carried out on a porous support composed of two or more layers:
a) extracting a nucleic acid using a porous support layer;
b) performing a nucleic acid amplification reaction for a target nucleic acid using a porous support layer containing a reagent for nucleic acid amplification; and
c) hybridizing a nucleic acid probe with the product of the nucleic acid amplification reaction, allowing a nucleic acid precipitant to act on the hybrid that has been produced so as to form an aggregate, and detecting the target nucleic acid in a specimen with the use of the obtained aggregate
wherein
i) a primer and the other reagents for nucleic acid amplification are separately retained within different porous supports, or
ii) a polymerase and the other reagents for nucleic acid amplification are separately retained within different porous supports, or
iii) the polymerase, the primer, and the other reagents for nucleic acid amplification are separately retained within different porous supports,
and the porous supports are allowed to come into contact with each other when used upon amplification.

4. The nucleic acid analysis method according to claim 3, wherein the porous support contains a nucleic acid precipitant and/or a nucleic acid probe.

5. The nucleic acid analysis method according to any one of claims 1 to 4, wherein the polymerase in a dry state is retained within the porous supports.

6. The nucleic acid analysis method according to any one of claims 1 to 5, wherein the nucleic acid amplification reaction is a LAMP reaction.

7. The nucleic acid analysis method according to any one of claims 1 to 6, wherein the porous support is made of at least one material selected from the group consisting of filter paper, nylon membrane, cellulose ester, cellulose, nonwoven fabric, woven fabric, cotton, polyurethane, and sintered plastics.

8. The nucleic acid analysis method according to any one of claims 3 to 7, wherein the nucleic acid precipitant is at least one member selected from the group consisting of hydroxyapatite, polyethylenimine, protamine sulfate, poly-L-lysine, and diethylaminoethyl dextran.

9. A nucleic acid analysis system for consistently performing nucleic acid extraction, amplification, and detection, such system comprising two or more porous supports containing a reagent for nucleic acid amplification, wherein
i) a primer and the other reagents for nucleic acid amplification are separately retained within different porous supports, or
ii) a polymerase and the other reagents for nucleic acid amplification are separately retained within different porous supports, or
iii) the polymerase, the primer, and the other reagents for nucleic acid amplification are separately retained within different porous supports.

## Patentansprüche

1. Nukleinsäure-Analyseverfahren umfassend das Zusetzen einer Nukleinsäure-haltigen Probe zu einem porösen Träger, der ein Reagenz zu Nukleinsäureamplifikation enthält, und eine Zielnukleinsäure einer Nukleinsäure-Amplifikationsreaktion unterziehen, wobei
a) ein Primer und die sonstigen Reagenzien für die Nukleinsäureamplifikation getrennt voneinander in unterschiedlichen porösen Trägern vorgehalten werden, oder
b) eine Polymerase und die sonstigen Reagenzien für die Nukleinsäureamplifikation getrennt voneinander in unterschiedlichen porösen Trägern vorgehalten werden, oder
c) die Polymerase, der Primer und die sonstigen Reagenzien für die Nukleinsäureamplifikation getrennt voneinander in unterschiedlichen porösen Trägern vorgehalten werden,
und die porösen Träger beim Einsatz bei der Amplifikation miteinander in Berührung kommen dürfen.

2. Nukleinsäure-Analyseverfahren nach Anspruch 1, umfassend das stetige Durchführen einer Nukleinsäureextraktion an der Probe, einer Amplifikationsreaktion für die Zielnukleinsäure und der Detektion der Nukleinsäure-Amplifikationsreaktion oder des Produkts derselben an dem porösen Träger.

3. Nukleinsäure-Analyseverfahren, das die folgenden Schritte umfasst, die an einem aus zwei oder mehr Schichten zusammen gesetzten porösen Träger durchgeführt werden:
a) Extrahieren einer Nukleinsäure mit Hilfe einer porösen Trägerschicht,
b) Durchführen einer Nukleinsäure-Amplifikationsreaktion für eine Zielnukleinsäure mit Hilfe einer porösen Trägerschicht, die ein Reagenz für die Nukleinsäureamplifikation enthält, und
c) Hybridisieren einer Nukleinsäuresonde mit dem Produkt der Nukleinsäure-Amplifikationsreaktion, Einwirkenlassen eines Nukleinsäure-Fällungsmittels auf das so hergestellte Hybrid, so dass ein Aggregat gebildet wird, und Detektieren der Zielnukleinsäure in einer Probe mit Hilfe des erhaltenen Aggregats,
wobei
i) ein Primer und die sonstigen Reagenzien für die Nukleinsäureamplifikation getrennt voneinander in unterschiedlichen porösen Trägern vorgehalten werden, oder
ii) eine Polymerase und die sonstigen Reagenzien für die Nukleinsäureamplifikation getrennt voneinander in unterschiedlichen porösen Trägern vorgehalten werden, oder
iii) die Polymerase, der Primer und die sonstigen Reagenzien für die Nukleinsäureamplifikation getrennt voneinander in unterschiedlichen porösen Trägern vorgehalten werden,
und die porösen Träger beim Einsatz bei der Amplifikation miteinander in Berührung kommen dürfen.

4. Nukleinsäure-Analyseverfahren nach Anspruch 3, wobei der poröse Träger ein Nukleinsäure-Fällungsmittel und/oder eine Nukleinsäuresonde enthält.

5. Nukleinsäure-Analyseverfahren nach einem der Ansprüche 1 bis 4, wobei die Polymerase in einem trockenen Zustand in den porösen Trägern vorgehalten wird.

6. Nukleinsäure-Analyseverfahren nach einem der Ansprüche 1 bis 5, wobei es sich bei der Nukleinsäure-Amplifikationsreaktion um eine LAMP-Reaktion handelt.

7. Nukleinsäure-Analyseverfahren nach einem der Ansprüche 1 bis 6, wobei der poröse Träger aus mindestens einem Material besteht, das aus der Gruppe ausgewählt ist, die aus Filterpapier, Nylonmembran, Celluloseester, Cellulose, Vliesstoff, Webware, Baumwolle, Polyurethan und gesinterten Kunststoffen besteht.

8. Nukleinsäure-Analyseverfahren nach einem der Ansprüche 3 bis 7, wobei das Nukleinsäure-Fällungsmittel als mindestens ein Mitglied der Gruppe ausgewählt wird, die aus Hydroxylapatit, Polyethylenimin, Protaminsulfat, Poly-L-Lysin und Diethylaminoethyl-Dextran besteht.

9. Nukleinsäure-Analyseverfahren zur stetigen Durchführung von Nukleinsäureextraktion, -amplifikation und -detektion, wobei das System zwei oder mehr poröse Träger aufweist, die ein Reagenz für die Nukleinsäureamplifikation enthalten, wobei
i) ein Primer und die sonstigen Reagenzien für die Nukleinsäureamplifikation getrennt voneinander in unterschiedlichen porösen Trägern vorgehalten werden, oder
ii) eine Polymerase und die sonstigen Reagenzien für die Nukleinsäureamplifikation getrennt voneinander in unterschiedlichen porösen Trägern vorgehalten werden, oder
iii) die Polymerase, der Primer und die sonstigen Reagenzien für die Nukleinsäureamplifikation getrennt voneinander in unterschiedlichen porösen Trägern vorgehalten werden.

## Revendications

1. Procédé d'analyse d'acide nucléique comprenant d'ajouter un échantillon contenant un acide nucléique à un support poreux comprenant un réactif pour l'amplification de l'acide nucléique et de soumettre un acide nucléique cible à une réaction d'amplification d'acide nucléique, dans lequel
a) une amorce et les autres réactifs pour l'amplification de l'acide nucléique sont fixés séparément à l'intérieur de différents supports poreux, ou
b) une polymérase et les autres réactifs pour l'amplification de l'acide nucléique sont fixés séparément à l'intérieur de différents supports poreux, ou
c) la polymérase, l'amorce et les autres réactifs pour l'amplification de l'acide nucléique sont fixés séparément à l'intérieur de différents supports poreux,
et les supports poreux sont mis en contact les uns avec les autres lorsqu'ils sont utilisés après l'amplification.

2. Procédé d'analyse d'acide nucléique selon la revendication 1, comprenant d'effectuer de manière régulière une extraction d'acide nucléique à partir de l'échantillon, une réaction d'amplification pour l'acide nucléique cible, et la détection de la réaction d'amplification de l'acide nucléique ou le produit de celle-ci sur le support poreux.

3. Procédé d'analyse d'acide nucléique, comprenant les étapes suivantes effectuées sur un support poreux composé de deux couches ou plus :
a) extraire un acide nucléique en utilisant une couche de support poreuse ;
b) effectuer une réaction d'amplification d'acide nucléique pour un acide nucléique cible en utilisant une couche de support poreux contenant un réactif pour l'amplification de l'acide nucléique ; et
c) hybrider un échantillon d'acide nucléique avec le produit de la réaction d'amplification de l'acide nucléique faisant agir un précipitant d'acide nucléique sur l'hybride qui a été produit de façon à former un agrégat, et détecter l'acide nucléique cible dans un échantillon en utilisant l'agrégat obtenu,
dans lequel
i) une amorce et les autres réactifs pour l'amplification de l'acide nucléique sont fixés séparément à l'intérieur de différents supports poreux, ou
ii) une polymérase et les autres réactifs pour l'amplification de l'acide nucléique sont fixés séparément à l'intérieur de différents supports poreux, ou
iii) la polymérase, l'amorce et les autres réactifs pour l'amplification de l'acide nucléique sont fixés séparément à l'intérieur de différents supports poreux,
et les supports poreux sont mis en contact les uns avec les autres lorsqu'ils sont utilisés après l'amplification.

4. Procédé d'analyse d'acide nucléique selon la revendication 3, dans lequel le support poreux contient un précipitant d'acide nucléique et/ou un échantillon d'acide nucléique.

5. Procédé d'analyse d'acide nucléique selon l'une quelconque des revendications 1 à 4, dans lequel la polymérase dans un état sec est fixée à l'intérieur des supports poreux.

6. Procédé d'analyse d'acide nucléique selon l'une quelconque des revendications 1 à 5, dans lequel la réaction d'amplification d'acide nucléique est une réaction LAMP.

7. Procédé d'analyse d'acide nucléique selon l'une quelconque des revendications 1 à 6, dans lequel le support poreux est fait d'au moins un matériau sélectionné parmi le groupe consistant en du papier-filtre, une membrane de nylon, de l'ester de cellulose, de la cellulose, du tissu non-tissé, du tissu tissé, du coton, du polyuréthane et des plastiques frittés.

8. Procédé d'analyse d'acide nucléique selon l'une quelconque des revendications 3 à 7, dans lequel le précipitant d'acide nucléique est au moins un élément sélectionné parmi le groupe consistant en l'hydroxyapatite, la poly-éthylènimine, le sulfate de protamine, le poly-L-lysine et le diéthylaminoéthyl-dextrane.

9. Système d'analyse d'acide nucléique pour d'effectuer de manière régulière une extraction, amplification et détection d'acide nucléique, un tel système comprenant deux ou plusieurs supports poreux contenant un réactif pour l'amplification de l'acide nucléique, dans lequel
i) une amorce et les autres réactifs pour l'amplification de l'acide nucléique sont fixés séparément à l'intérieur de différents supports poreux, ou
ii) une polymérase et les autres réactifs pour l'amplification de l'acide nucléique sont fixés séparément à l'intérieur de différents supports poreux, ou
iii) la polymérase, l'amorce et les autres réactifs pour l'amplification de l'acide nucléique sont fixés séparément à l'intérieur de différents supports poreux.
